(19) Europäisches Patentamt / European Patent Office / Office européen des brevets

(11) EP 4 574 985 A1

(12) EUROPEAN PATENT APPLICATION

(43) Date of publication:
**25.06.2025 Bulletin 2025/26**

(21) Application number: **23383360.7**

(22) Date of filing: **22.12.2023**

(51) International Patent Classification (IPC):
***C12P 7/649*** *(2022.01)* ***C10L 1/02*** *(2006.01)*
***C12P 7/6458*** *(2022.01)* ***C12P 17/04*** *(2006.01)*
***C12P 17/06*** *(2006.01)*

(52) Cooperative Patent Classification (CPC):
**C12P 17/04; C10L 1/02; C10L 1/026; C12P 7/6458; C12P 7/649; C12P 17/06**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Selabtec Sciences, S.L.**
**08225 Barcelona (ES)**

(72) Inventors:
- **HERRERO DÁVILA, Lorenzo**
  **08225 BARCELONA (ES)**
- **BAYARRI FERRER, Natividad**
  **08225 BARCELONA (ES)**

(74) Representative: **Herrero & Asociados, S.L.**
**Edificio Aqua - Agustín de Foxá, 4-10**
**28036 Madrid (ES)**

# (54) A PROCESS FOR PREPARING A BIOFUEL COMPOSITION, THE BIOFUEL COMPOSITION OBTAINABLE BY THE PROCESS AND THE USE OF THE SAME

(57) The present invention relates to a process for preparing a biofuel composition, the biofuel composition obtainable by the process and the use of the biofuel. More specifically, the present invention is related to a process for producing, biofuel defined by a blend of fatty acid formal glycerol ester (FAGE) /fatty acid alkyl ester/acetal mixture/hydrotreated vegetable oil (HVO), the process being characterized by an enzymatic transesterification of fatty acid alkyl esters with glycerol formal and by the specific rates of the different components. The invention is also directed to the biofuel compositions obtained by said process as well as to the uses thereof.

EP 4 574 985 A1

## Description

### FIELD OF THE INVENTION

The present invention relates to a process for preparing a biofuel composition, the biofuel composition obtainable by the process and the use of the biofuel. More specifically, the present invention is related to a process for producing biofuel, defined by a blend of fatty acid formal glycerol ester (FAGE) /fatty acid alkyl ester/acetal mixture/hydrotreated vegetable oil (HVO), the process being characterized by an enzymatic transesterification of fatty acid alkyl esters with glycerol formal and by the specific rates of the different components. The invention is also directed to the biofuel compositions obtained by said process as well as to the uses thereof.

### BACKGROUND OF THE INVENTION

Biofuel is a type of renewable energy derived from organic materials, such as plants, agricultural crops, or biological waste. Biofuel is a broad term encompassing any fuel derived from organic materials, including biodiesel. Biodiesel is a specific type of biofuel that is made from renewable biological sources, such as vegetable oils, animal fats, or recycled cooking oils. While biofuels can include a range of renewable fuels like ethanol, biogas, and others, biodiesel specifically refers to a diesel-like fuel that is produced from biological sources. The production process for biodiesel involves the transformation of triglycerides (found in oils and fats) through transesterification, resulting in a fuel that can be used in diesel engines.

The main advantage for the use of this biofuel is the overall reduction of particle and $NO_x$, in addition to decreased carbon footprint, lower toxicity and being readily biodegradable than petroleum diesel.

Reduction of the sources of crude oil and increasing concern on noxious gas emissions have encouraged legislative and regulatory proposals to replace petroleum fuels with other fuels obtained from renewable sources.

This biofuel performs similar to petroleum-diesel, but their properties are different. It is important that the biofuel shows a good lubricity and adequate cetane range.

Hydrogenated vegetable oil (HVO) is a renewable diesel which can be produced from various vegetable oils and fats. There are some properties of HVO that may limit its capability to replace diesel fuels: the high cetane number, or propensity to auto ignite, is very high. The lubricity is very low due to the absence of sulfur and oxygen compounds in the fuel.

EP2730567 shows, for instance, a process for simultaneously producing alkyl esters of fatty acids fatty acids, glycerol formal and FAGE. The chemical reaction disclosed in EP2730567 is mediated by a Titanium catalyst, thus not being an enzymatically driven reaction.

EP2049623 also discloses a chemical process for producing FAGE from rapeseed oil but, again, it does not teach the use of lipolytic enzymes to this end.

WO2014102796 describes a process and a reactor for transesterification with immobilized enzymes in the production of fatty acid alkyl esters for use, *inter alia,* as biofuels. According to this document, the fatty acid alkyl esters are FAME (fatty acid methyl esters) or FAEE (fatty acid ethyl esters).

Therefore, it is necessary to develop new processes for preparing new biofuels with improved behaviour and also prevailing technical standards in the biofuel sector.

### SUMMARY OF THE INVENTION

The improvement of the biofuel has been addressed by blending a FAME/FAGE biodiesel with acetal mixtures and HVO. Blending strategies are useful for example to improve features like lubricity, cetane number, cold flow properties, essential features to develop a good biofuel composition. In the present invention has been development a process to obtain a blending to optimize the biodiesel performance and emissions. The features of the biofuel composition of the invention lead to benefits on reduction of carbon monoxide, total hydrocarbon and more significantly in particulate engine emissions.

The process herein described, entails the conversion of fatty acid alkyl esters into fatty acid glycerol formal esters (FAGE) via enzymatic transesterification as means to a chemical incorporation of the molecule of glycerol (main component of crude glycerin, industrial waste from biodiesel production) into a high value chemical product. The present patent improves the state of the art by incorporating said FAME/FAGE as a component in mixtures of renewable hydrocarbons and, therefore, is an effective solution to develop a new generation of more sustainable biofuels. FAME is concomitantly obtained during the manufacturing process of FAGE in different proportions depending of the reaction conditions. Since FAGE cold flow properties are in disadvantage when compared to FAME, the influence of some cold-flow improvers in FAGE/FAME blends has been development. An important novel aspect of this invention is the use of lipolytic enzymes as transesterification catalysts and in its immobilized forms. These have advantages from the process efficiency

and recovery point of view for this process. The use of enzymes in this process is a flexible, efficient and cleaner strategy that the chemical processing previously reported.

The mentioned ester product it is by itself, unsuitable to meet international quality fuel standards for its direct use in diesel engines, therefore the processing steps here described, are required to allow FAGE to become part of, fully renewable biofuels for its use in diesel engines. In the present process are essential features the addition of an acetal mixture of glycerol formal/methoxy methyl glycerol formal (GFoMoM) (step d) and hydrotreated vegetable oil (HVO), compounds that improve the behavior of the biofuel, for example, improving cold flow, viscosity and stability properties. The biofuel obtained meet international quality fuel standard.

Therefore, the first aspect of the invention is a process for preparing a biofuel composition comprising the steps of:

a) reacting a fatty acid alkyl ester of formula (II) or a source of fatty acid alkyl esters of formula (II) with glycerol formal in the presence of an immobilised lipolytic enzyme,

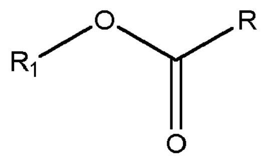

**(II)**

to obtain fatty acid glycerol formal ester (FAGE) of formula (I):

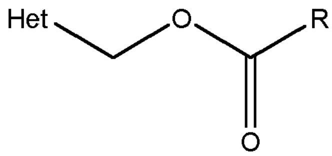

**(I)**

b) neutralizing excess fatty acids to acid index below 0.5mg KOH/g of sample;

c) adding a fatty acid alkyl ester of formula (II) to obtain a ratio of fatty acid alkyl esters /FAGE from 60/40 to 90/10 wt.%;

d) adding an acetal mixture of glycerol formal/ methoxy methyl glycerol formal (GFoMoM) in a ratio from 1 to 5 v/v.% respect to the total volume of fatty acid alkyl esters /FAGE resulting an ester/acetal mixture;

e) adding hydrotreated vegetable oil (HVO) to obtain a ratio (v/v.%), HVO/the ester/acetal mixture obtained in d) from 60/40 to 90/10;

where

Het is selected from;

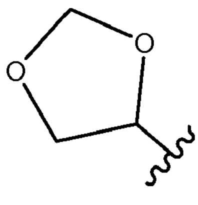

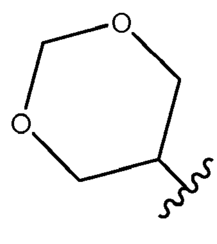

**R** $C_1$-$C_{28}$ alkyl radical or alkenyl radical $C_1$-$C_{28}$ ; y
**$R_1$** $C_1$-$C_6$ alkyl radical.

The term "biofuel" means a solid, gas or liquid fuel derived entirely from biomass that can be used as a replacement of fossil fuel and for the case of gases and liquids mainly in automotive applications.

The term "Fatty acid glycerol formal Ester" or "FAGE": means esters of fatty acids resulting from the esterification of a certain fatty acid (generally $C_1$-$C_{28}$) with glycerol formal. In the context of the invention, it is produced by transesterification of fatty acid alkyl esters, preferably FAME, with glycerol formal.

The term "Fatty acid alkyl Ester" means esters of fatty acids resulting, in the presence of a catalyst, from the esterification reaction of a free fatty acid (generally $C_1$-$C_{28}$) and an alcohol, or from the transesterification reaction of triglyceride also, producing glycerol as co-product.

The term "Source of fatty acid alkyl esters" means raw material, generally of natural origin, where fatty acid alkyl esters can be derived from, such as animal fats, industrial wastes and miscellaneous vegetable oils. The term "Animal fats" means lipid fractions extracted from the processing animal matter, which mayor components are triglycerides and free fatty acids. Animal fats are usually solid at room temperature. The term "Vegetable oils" means lipid fractions extracted from the processing seeds or vegetable matter, which mayor components are triglycerides and free fatty acids. Vegetable oils are usually liquid at room temperature. The term industrial waste means lipid fractions or derivatives extracted or obtained from the processing of Vegetable oils and Animal fats, such but nor exclusively crude glycerine.

The term "$C_1$-$C_{28}$ alkyl radical": linear or branched, preferably linear, saturated aliphatic radicals containing from 1 to 28 carbon atoms.

The term "$C_1$-$C_{28}$ alkenyl radical": linear or branched, preferably linear, unsaturated aliphatic radicals containing from 1 to 28 carbon atoms and having from 1 to 7 double bonds.

The term "Lipolytic enzyme" means lipolytic enzymes are a genetically varied group of industrially or non-industrial enzymes capable of catalyzing both the hydrolysis and the synthesis of lipids as well as esterification and transesterification reactions in lipidic substrates. In the context of the present invention the relevant lipolytic enzymes are those with capacity for transforming by transesterification reaction fatty acid alkyl ester, preferably FAME, into FAGE.

The term "Immobilized enzyme" means enzyme attached to an inert, insoluble material, in the reaction medium. In this way, it provides to the enzyme an increased mechanical resistance or to changes in temperature, acidity or pH. Inmmobilized enzymes remain in place throughout the reaction and facilitates their recovery and re-usage.

The term glycerol formal (GF), CAS number 99569-11-6 means a mixture of mixture of 1,3-dioxan-5-ol and 1,3-dioxolan-4-ylmethanol, in this invention, derived but not exclusively from crude glycerine

The term GFoMoM means methoxy methyl glycerol formal. This product can be generated in the transacetalisation reaction and is represented as follows:

Scheme 1

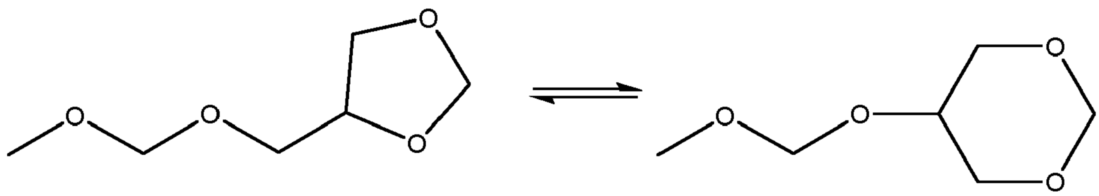

The biofuel composition obtainable in the present invention has an adequate lubricity, reduction of total hydrocarbon and reduction of particle emissions.

Therefore, the second aspect of the invention is a biofuel composition obtainable by the process of the invention. The biofuel composition defined in the second aspect of the invention is a biofuel without mineral diesel.

The biofuel composition of the invention is suitable to be employed in diesel engines with international quality standards. The biofuel of the invention shows optimum parameter for density, viscosity and cold flow performance to be use in diesel engines. Also, the particle emissions are minimum.

Finally, the biofuel, obtained could be used as fuel for diesel-powered vehicles, meeting the parameters established by the community standard. The biofuel composition is suitable to be employed in diesel engines as part of fully renewable diesel compositions, for subsequent compliance with international quality standards for the use of fuels in diesel engines.

Therefore, the third aspect of the invention is the use of the biofuel composition according the second aspect of the invention as fuel for diesel engines. This use without the need of blending with mineral diesel.

The term diesel means types of fuels of defined specifications generally employed in internal combustion engines for automotive or of for heating purposes.

## DETAILED DESCRIPTION OF THE INVENTION

As it is mentioned above the first aspect of the invention refers to a process for preparing a biofuel composition comprising the steps of:

a) reacting a fatty acid alkyl ester of formula (II) or a source of fatty acid alkyl esters of formula (II)

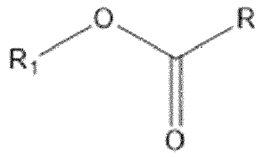

**(II)**

with glycerol formal in the presence of an immobilised lipolytic enzyme, to obtain fatty acid glycerol formal ester (FAGE) of formula (I):

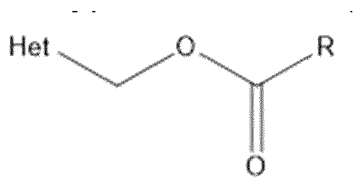

**(I)**

b) neutralizing excess fatty acids to acid index below 0.5mg KOH/g of sample.

c) adding a fatty acid alkyl ester of formula (II) to obtain a ratio of fatty acid alkyl esters /FAGE from 60/40 to 90/10 wt.%;

d) adding an acetal mixture of glycerol formal/ methoxy methyl glycerol formal (GFoMoM) in a ratio from 1 to 5 v/v.% respect to the total volume of fatty acid alkyl esters /FAGE resulting an ester/acetal mixture;

e) adding hydrotreated vegetable oil (HVO) to obtain a ratio (v/v.%), HVO/the ester/acetal mixture obtained in d) from 60/40 to 90/10;

where

Het is selected from;

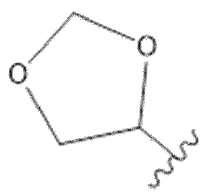

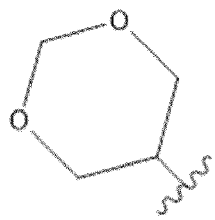

R $C_1$-$C_{28}$ alkyl radical or alkenyl radical $C_1$-$C_{28}$; and
$R_1$ $C_1$-$C_6$ alkyl radical.

Preferably the step a) is carried out at temperatures between 30-70°C, more preferably 35-65° and even more preferably between 45-65°C.

Preferably the steps are carried out under stirring.

In a particular and preferred embodiment, $R_1$ represents a methyl group resulting in that the fatty acid alkyl ester

used as substrate for the reaction is a fatty acid methyl ester (FAME) of formula (IIa):

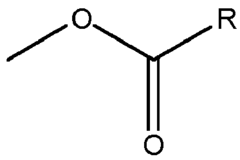

**(IIa)**

wherein R is as defined above.

The reaction between the compound of particularly preferred compound of formula (IIa) and glycerol formal according to the invention is schematically represented in scheme 1 below:

**Scheme 2**

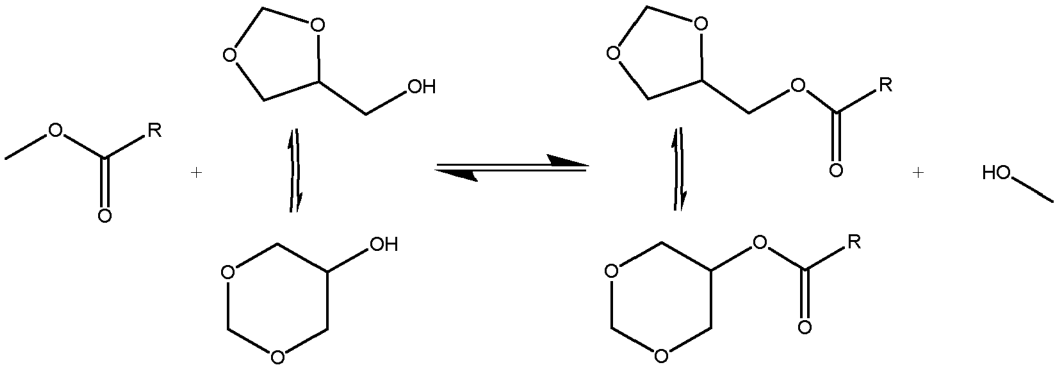

**1 FAME** **1 GF** **1 FAGE** **1 METHANOL**

The substrate of the reaction can be a purified mixture of fatty acid alkyl esters of formula (II), preferably of formula (IIa) or a source of fatty acid alkyl esters of formula (II) and/or of formula (IIa). Any lipidic source of fatty acid alkyl ester of formula (II) and/or of formula (IIa) is useful as a substrate for reaction in the process of the invention, although in a preferred embodiment the source of fatty acid alkyl esters of formula (II) and/or of formula (IIa) comprises at least one of animal fats, vegetable oils, food wastes, used cooking-oils, non-food crops, algae oils, oleochemical and industrial residues or mixtures thereof. The possibility of using any of these sources of fatty acid alkyl esters has the advantage of giving the process of the invention a great versatility and the possibility for reusing and recycling waste materials to give a product with an important added value.

The term "Fatty acid methyl ester" or "FAME" means a fatty acid alkyl ester where the alkyl group is a methyl.

The inventors however have found that when the lipolytic enzyme was selected from *Thermomyces lanuginosus* lipase, *Candida antarctica* lipase B, *Proteus sp.* K107 Lipase or a mixture thereof, the reaction was not only very selective towards FAGE production but, in addition, yielded a reaction product with a high proportion of FAGE. Therefore, preferably the the lipolytic enzyme was selected from *Thermomyces lanuginosus* lipase, *Candida antarctica* lipase B, *Proteus sp.* K107 Lipase or a mixture thereof.

The term *"Thermomyces lanuginosus* lipase" in the present invention refers to the natural or modified lipolytic enzyme sourced from the thermophilic fungus *Thermomyces lanuginosus.*

The term *"Candida antarctica* lipase B" in the present invention refers to the natural or modified lipolytic enzyme sourced from the yeast *Candida antarctica.*

The term *"Proteus sp. K107* lipase" in the present invention refers to the natural or modified lipolytic enzyme sourced from the bacteria *Proteus sp.*

For optimizing the reaction, the loading of the lipolytic enzyme or mixture of lipolytic enzymes in the reaction must be preferably between 2-20 wt.% by weight, preferably 2-10 wt.% by weight, more preferably between 2-5 wt.% by weight with respect to the fatty acid alkyl ester substrate.

The present invention offers an advantage in the described biofuel, wherein the range of the raw materials from which FAME/FAGE is derived is broad. This expansive scope of feedstock enhances the versatility and sustainability of the biofuel process.

Preferably the FAME and FAGE are derived from animal fats, rapeseed oil, food waste oil, used cooking oil, sunflower oil, palm oil, coconut oil, soy oil, olive oil, industrial residues and mixtures thereof. More preferably step b) is neutralizing excess fatty acids to acid index between 0.1-0.5mg KOH/g of sample.

Preferably in the step c) the ratio fatty acid alkyl esters /FAGE is from 70/30 to 80/20 wt.%. Preferably in the step d) adding the acetal mixture glycerol formal/methoxy methyl glycerol formal (GFoMoM) in a range from 2 to 4 v/v.% to the total volume of ester product from c). Preferably, in step e) the ratio HVO/the ester/acetal mixture obtained in d) from 65/35 to 80/20 v/v.%.

Preferably the ratio in the acetal mixture glycerol formal/GFoMoM is between 50/50 to 90/10 wt.%. More preferably the process comprises a step previous to step d) of mixing the acetal mixture glycerol formal/GFoMoM between 50/50 to 90/10 wt.% with an alkali metal hydride dissolved in a solvent and filtering and further distil it to obtain an acetal mixture glycerol formal/GFoMoM between 30/70 to 10/90 wt.%. More preferably the solvent is an aprotic solvent, particularly THF (tetrahydrofuran).

The "$C_1$-$C_{28}$ alkyl radical" term means linear or branched, preferably linear, saturated aliphatic radicals containing from 1 to 28 carbon atoms. More preferably containing 1 to 10 carbon atoms.

The "$C_1$-$C_{28}$ alkenyl radical" term means linear or branched, preferably linear, unsaturated aliphatic radicals containing from 1 to 28 carbon atoms and having from 1 to 7 double bonds. More preferably containing 1 to 10 carbon atoms.

Preferably the process comprises adding additives selected from the group of antioxidants and cold flow improvers and/or antifoaming agents in step d) or after step d).

The other aspect of the invention is the biofuel composition obtainable by the process of the invention. Preferably the biofuel composition shows a density value between 765 kg/m3-845 kg/m3 measured by EN ISO 3675. More preferably the biofuel shows a kinematic viscosity value between 2 $mm^2/s$-4.5 $mm^2/s$ measured by EN ISO 3104. In other more preferably embodiment, the biofuel composition has a cold filter plugging point lower than 5°C measured by EN 116.

Examples of the invention

Example 1

A FAME/FAGE composition was obtained from a used cooking oil and animal fat (UCO/AF)-derived FAME ((A), 7.3Kg), through an enzymatic transesterification, reacting glycerol formal, (GF). The crude ester mixtures (B) were stirred and treated with an aqueous solution of 10 wt.% KOH in water at ambient temperature to correct acidity. The soap phase was decanted and removed. The alkalinity then washed off with sequential water washes up to pH in those, dropped to values of 6-7. Remaining soaps from the washed crude were removed by a fritted filter and the filtered phase was evaporated at 45°C below 15 mbar for a period of 16 hours and set for recovery. The dried ester obtained was passed through a cellulose filter (100□m) and the purified FAME/FAGE mixture (C) isolated at a yield of 77.9 wt.%. (C) was then mixed with an additional amount of FAME (A) from the same batch, to adjust FAME/FAGE proportion the nearest to the optimum 70/30 wt.%. In addition, a proportion of 3 v/v.% of the acetal mixture glycerol formal/GFoMoM was added to the 70/30 wt.%-FAME/FAGE composition (C) plus 1000 ppm of butylated hydroxytoluene (BHT) to generate the product Fuel (D), providing it with improved cold flow, viscosity and stability properties over (B and C). The Fuel (D) was tempered for two hours at 40°C and stirred while blending in a proportion 80/20 v/v.% Fuel (D ) with Hydrotreated Vegetable Oil (HVO).

The acetal mixture glycerol formal/GFoMoM was obtained from other acetal mixture of composition 58 wt.%, and 34 wt.%, in GF and GFoMoM respectively.

1200 ml of THF are loaded into a spherical reactor, innertized with nitrogen splurging and vigorous for a minimum period of 30 minutes and then 150g of NaH dissolved in the solvent.

600g of the dried acetal mixture were loaded into an addition funnel and once the NaH is fully dissolved, the acetal mixture is slowly added to the hydride solution in order to keep the reaction mixture below 15°C.

Once the addition completed, the reaction mixture was left stirring overnight at room temperature. The crude was filtered, the GF-salt kept aside and the THF was evaporated under vacuum. More GF-salt precipitates filtered and washed with THF.

The liquid filtered was distilled under vacuum at 80°C, and (A) GFoMoM was obtained. (yield 70.0 wt.%, purity 84 wt.%).

A subsequent comparison between the biodiesel composition of this Example 1 (E), HVO and diesel specifications is made in the following table.

Table 1. Properties of biodiesel composition of the invention (E), HVO and diesel.

| Property | Biodiesel composition of the Example 1 (E) | HVO | Diesel | Method |
|---|---|---|---|---|
| | Average | Average | Average | |
| Density at 15°C (kg/m$^3$) | 808.73 | 779.33 | 826.6 | EN ISO 3675 |
| Kinematic viscosity at 40°C (cSt) | 3.764 | 2.772 | 2.585 | EN ISO 3104 |
| Higher calorific power (MJ/kg) | 45.2484 | 47.3630 | 45.799 | UNE 51123 |
| lower calorific value (MJ/kg) | 42.370 | 44.030 | 42.895 | UNE 51123 |
| Derivative cetane number | 69.07 | 72.72 | 59.85 | EN 16715 |
| Wear track diameter ($\mu$m) | 251.3 | 259.6 | 429 | EN ISO 12156-1 |
| Water content (ppm) | 94.1 | 35.6 | 40 | EN ISO 12937 |
| Oxidation stability (h) | > 48 | - | - | EN 15751 |
| Elemental Analysis C (wt.%) | 82.32 | 84.7 | 86.41 | - |
| Elemental Analysis H (wt.%) | 13.15 | 15.3 | 13.59 | - |
| Elemental Analysis O (wt%) | 4.53 | - | - | - |
| Smoke point (mm) | 36.47 | 34.53 | 22.97 | ASTM D 1322-97 |
| OESI/TSI | 16.20 | 15.295 | 14.33 | |
| Cold filter plugging Point (°C) | -10 | -31 | -23 | EN 116 |
| Molecular weight (g/mol) | 231.5 | 221.6 | 203.08 | - |
| Stoichiometric dosing | 1/13.74 | 1/14.88 | 1/14.49 | - |

Example 2

A rapeseed/Animal Fat derived FAME (F) blend (1001.7g), was reacted with (407.3g) of GF 98 wt%, in the presence of Novozyme 435 (30g) under vacuum (below 70mbar) agitation (200rpm) and nitrogen flow at 60°C, for a total reaction time of 5 hours. The remaining GF in the reaction crude was distilled under vacuum and nitrogen flow, followed by a filtering step to remove any solid impurity in the crude ester. The composition (G) was obtained. Acidity and the remaining properties were corrected by blending with (F) to lead to (H).

Further addition of 3 v/v.% of the acetal mixture glycerol formal/GFoMoM and 1000 ppm of BHT yields the product (I).

(I) was tempered at 40°C for two hours and subsequently blended with Hydrotreated Vegetable Oil (HVO from Table 2) in proportions 20-35/80-65 v/v% product (I)/ HVO respectively.

Density, viscosity and CFPP for this composition of the invention was compared with the requirements from the EN590 (diesel) and EN15940 (HVO) standards. (Table 2).

Table 2 Properties of the biofuel composition of the invention (J) obtained in Example 2.

| | Viscosity (cSt) 40 °C Method: EN ISO 3104 | | EN590 | EN15940 | |
|---|---|---|---|---|---|
| Proportion product (I)/HVO (v/v.) | Example 1 (Product E) | Average value | Specification | Class A Specification | Class B Specification |
| 20% | 3.764 | | 2.0-4.5 | 2.0-4.5 | 2.0-4.5 |
| 25% | | 3.661 | | | |
| 30% | | 3.707 | | | |
| 35% | | 3.892 | | | |
| | Density (Kg/m3) 15°C | | | | |

(continued)

| | Viscosity (cSt) 40 °C Method: EN ISO 3104 | | EN590 | EN15940 | |
|---|---|---|---|---|---|
| Proportion product (I)/HVO (v/v.) | Example 1 (Product E) | Average value | Specification | Class A Specification | Class B Specification |
| | Method: EN ISO 3675 | | | | |
| 0% | 808.73 | 765.86 | 820-845 | 765-800 | 780-810 |
| 20% | | | | | |
| 25% | | 796.19 | | | |
| 30% | | 805.78 | | | |
| 35% | | 812.73 | | | |
| 100% | | 892.93 | | | |
| | Cold flow plug point (CFPP. °C) Method:EN 116 | | | | |
| 0% | -10 | -33 | Compliant with cold winter zone Grade F | Compliant with cold winter zone Grade F | |
| 20% | | | Compliant with cold winter zone Grade D | Compliant with cold winter zone Grade D | |
| 25% | | -7 | Compliant with cold winter zone Grade C | Compliant with cold winter zone Grade C | |
| 30% | | -5 | Compliant with cold winter zone Grade C | Compliant with cold winter zone Grade C | |
| 35% | | -4 | Compliant with cold winter zone Grade B | Compliant with cold winter zone Grade B | |
| 100% | | 0 | Out of specification for EN590 | Out of specification for EN590 | |
| | Cloud point (°C) Method:EN 116 | | Parameter not listed in the norms. | | |
| 25% | | -7.40 | | | |
| 30% | | -6.40 | | | |
| 35% | | -5.17 | | | |

Example 3

Palm oil-derived FAME ((K) 1082g) of methyl ester content 97.7 wt%, was tempered at 40°C, and further reacted at 60°C in a batch reactor, using immobilized lipase (35.32g) as catalyst plus the sequential addition of a GF (total addition of glycerol formal 960g). A continues nitrogen flow was employed and vacuum (30-70mbar) for a total period of 15 hours.

After that time, unreacted GF was removed from the crude by distillation under nitrogen and vacuum leading a FAGE ester crude (L) with a content of 84 wt.% FAGE, low acidity and GF 1.8 wt%.

(L) FAGE was further blended with a certain amount of (K) to yield (M), a FAME/FAGE composition suitable for its use with hydrocarbon fuels. After the addition of 3 v/v. % of the acetal mixture glycerol formal/GFoMoM and 1000 ppm of BHT, (N) was obtained. The later product was used for blending with HVO.

Table 3 Properties of the biofuel composition (N) obtained in Example 3

| Proportion SLB·Fuel/HVO (v/v.) | Viscosity (cSt) 40 °C Method: EN ISO 3104 | Density (Kg/m3) 15°C Method: EN ISO 3675 | CFPP °C Method:EN 116 |
|---|---|---|---|
| | Average value | Average value | Average value |
| 35% | 3.725 | 825.4 | 3 |
| EN590 | 2.0-4.5 | 820-845 | Compliant with warm winter zone Grade A |
| EN 15940 | | 780-810 (Class B) | Compliant with warm winter zone Grade A |

Example 4. Use of the biofuel composition of the Example 1 in engine and vehicle tests

The composition obtained in the Example 1, was benchmarked in real engine test conditions versus, standard diesel and HVO, under standardized driving conditions. 5l of each fuel were employed for each trial in two different engine setups;

- An engine-bench.
- An engine in a roller bench in a real vehicle inside a climatic chamber;

Each of the facilities comprise of the following equipment:
Engine Bench. A Nissan K9K 646 1.5dCi engine four-stroke, four-cylinder in-line, supercharged, electronically governed common-rail injection, equipped with oxidation catalyst (DOC, Diesel Oxidation Catalyst), NOx (LNT, Lean NOx Trap) and particulate filter (DPF, Diesel Particulate Filter). This engine complies with Euro 6 regulations on polluting emissions.
The emissions were measured in a dilution system and EEPS TSI's 3090 Engine Exhaust Particle SizerTM Spectrometer (EEPS) is based on the measurement of the mobility of charged particles within an electric field, and is capable of measuring particle size distributions from 5.6 to 560 nm.

Description of the roller bench and climatic chamber

The roller bench used is located inside a climatic chamber that allows the temperature and humidity of the room to be set, which greatly increases the repeatability of the tests carried out. The car is anchored to the roller bench system, although it allows free movement of the vehicle front wheels'. The speed, the inertia of the car and acclimatization, are simulated by the combined operation of rollers and blowers, both responsible for absorbing the energy from the car wheels while moving, plus replicating the car's resistance to the wind and external temperature.
The procedure follows the guidelines given in WLTP, measuring the car speed in different driving circles with $\pm5$ km/h at speeds below 50 km/h and $\pm10$ km/h at higher speeds.
To guarantee a sufficient supply of air for the correct operation of the vehicle, the room is equipped with a fresh air renewal system, which dries before entering the test room.
If specific humidity conditions are desired, it can be moistened after drying. This air conditioning and air renewal system as a whole allows reaching temperature conditions from -20 °C to 40 °C, also allowing relative humidity control between 5% and 90% when the temperature is above 16 °C. The supervision and control of the air conditioning of the test room is carried out from an adjacent control station thanks to the SCADA software ("Supervisory Control and Data Acquisition") and a series of automatons distributed throughout the installation.

Engine in the vehicle

Peugeot 3008 It has 5 doors and 5 seats and belongs to the C-SUV segment, is powered by a supercharged four-cylinder four-stroke engine (code DV6 FC), with direct fuel injection compression ignition through the common-rail system and is equipped with an SCR catalytic converter as a NOx after-treatment system thanks to which is capable of complying with the limit for said emission established by the Euro 6b regulation, in force at the time of purchase. The engine is located at the front of the vehicle, with a transverse arrangement and is coupled to a 6-speed manual gearbox that is responsible for transmitting movement to the front wheels. Description of the polluting emission measurement equipment, cycle tracker and ECU.

To carry out the measurement of the polluting emissions of the vehicle during the driving cycles, several pieces of equipment have been used arranged along the exhaust line of the vehicle. It is important to point out that the measurement of particle size distribution is only carried out before the DPF, since these filters have retention efficiency greater than 99%.

One of the equipment used for the measurement of gaseous compounds has been the MEXA-ONE, which meets all the necessary requirements imposed by regulation No. 15 of the UNECE for the measurement of vehicle emissions. This equipment consists of two different cabinets or racks, the MEXA-ONE-C1 and the OVN-25H. The MEXA-ONE-C1 consists of several cells in which the measurement of CO, $CO_2$ and NOx is carried out, all of them on a cold and dry basis that is, previously eliminating the water that accompanies the exhaust gas. In the case of CO and $CO_2$, the equipment uses a non-dispersive infrared measurement principle or NDIR (Non-Dispersive InfraRed). For the measurement of $NO_x$, a chemiluminescence detector or CLD is used, for which it requires the generation of ozone from an $O_2$ supply. The other rack that makes it up, the OVN-25H, is used to carry out the NO and THC measurements, and unlike the MEXA-ONE-C1, it carries them out on a wet basis. It uses a CLD detector to measure, in this case, the NO present in the exhaust gas. Thanks to this, the concentration of $NO_2$ can be estimated by means of the difference between the NO and the total NOx measured by each of the available analyzers, previously converting from dry to wet base. The other analyzer found in the OVN-25H is a flame ionizer detector or FID (Flame Ionization Detector) that is used to measure the concentration of total hydrocarbons (THC) present in the exhaust gas, which is expressed in ppm. methane equivalents (ppmC). The measurement carried out by this FID occurs in a heated environment at 191 °C to avoid unwanted condensation.

To accurately reproduce the driving cycle, a cycle tracker is used, in which the speed profile to be reached by the driver of the vehicle is represented together with the upper and lower limits of error that can be made, which is ±2km/h The entire speed and emissions synchronization process is automated and managed by the STAR VETS software.

To sample the particles emitted by the engine in each of the vehicles, an EEPS (Engine Exhaust Particle Sizer) spectrometer model 3090 of the TSI brand has been used, which measures the size distribution of the exhaust gas particles (a similar installation to the employee and described in Report 2 of this provision of services). The exhaust gas sample cannot be introduced directly into the EEPS as the equipment could be damaged but must be conditioned according to temperature and dilution requirements. For this, a previous double dilution system is used that is divided into a rotating disk diluter (Rotating Disk diluteror RD) model MD19-2E, where the first dilution is carried out, and an ASET15-1 temperature conditioner equipped with an evaporator, where the second is carried out.

To record all these changes in the engine operating parameters during the tests, a connection to the ECU has been used through an ETAS ES592.1 module and the use of INCA PC software, also from the company ETAS Gmbh. With this, the aim is to detect if a variation in performance or emissions is due directly to the fuel itself or indirectly to changes in the operating parameters. Data acquisition is done via OBD-II connector.
The protocol followed has always been the one described below:

Maceration of the vehicle for 3 hours from the conditioning of the climatic room and the engine parts. Temperatures are checked at all times by means of the different sensors distributed around the room and the data provided by the vehicle's ECU.
Prior to each test, conditioning, calibration and zero of MEXA (gas emissions equipment) and zero of EEPS using HEPA filter.
Heating of roller bearings and electric machine. To do this, the vehicle's wheels are rotated, in neutral, at 80 km/h for 7 minutes (Warm-Up Mode).
Switching on heated lines for particle measurement.

Between cycles:

At the end of each test, the vehicle battery is recharged using an external charger connected to the network.
Cleaning of the entire dilution system.
Checking the vehicle's anchorages

Between fuels:
Complete bleeding of the tank and the fuel supply system.

All tests with the three fuels used (Diesel, HVO and Biodiesel obtained in Example 1) are tested at least three times, showing average values. Assays are performed at 23°C and at -7°C.

Table 4 show the differences of the biofuel of Example 1 with respect to diesel and HVO in the K9K engine (bars with lines) and in the Peugeot 3008 vehicle (solid bars) for tests carried out under ambient conditions.

The main result obtained is that there is no significant difference in consumption when replacing diesel/HVO with the biofuel composition of the invention.

The most relevant differences appear with particulate emissions. The biofuel composition of the present invention reduces these emissions, as well as those of carbon monoxide, with respect to both diesel and HVO for both

engine settings.

The variations in hydrocarbons are not relevant due to their low levels (very close to the detection limit of the equipment used).

Table 4 Summary of fuel performance for the biofuel of the Example 1

| | Fuel consumption (g) | Particles (#) | Particles (g) | CO (g) | $CO_2$ (g) | $NO_x$ (g) | HC (g) |
|---|---|---|---|---|---|---|---|
| Compared to HVO (3008) | 2% | -43% | -56% | -23% | -1% | 32% | -19% |
| Compared to diesel (3008) | -1% | -65% | -85% | -35% | -5% | 23% | 6% |
| Compared to HVO (K9K) | 7% | -80% | -83% | -17% | -2% | 6% | 0% |
| Compared to diesel (K9K) | 2% | -83% | -83% | -36% | -5% | 11% | -23% |

Example 5. Selection of lipolytic enzymes for the synthesis of glycerol formal fatty acid esters

Different commercial and non-commercial lipases (see Table 5) were screened for investigating the activity for the production of FAGE. Enzymatic transesterification reactions were performed using 800 μl of FAME (2.7mmol), 200 μl of GF (2.3mmol), 50 μl of water, and 3 wt.% of biocatalyst. The reactions were carried out at 40°C, using the multipoint reactor Carrusel 12 plus reaction station (Radleys, Saffron Walden, UK). Reactions were conducted in 50 mL reaction tubes continuously mixed using a magnetic stirrer (200 rpm), and under vacuum in order to remove the methanol formed during the reaction, which shifted the reaction toward synthesis. To obtain the time course of the reaction, 50 μL of sample were taken at different time points and then analyzed via gas chromatography (GC-FID).

FAME and FAGE content were estimated by a correlation of area by using in this case, methyl heptadecanoate as internal standard following an adapted method from EN14103. The direct correlation between mass and response, with identical response factor for every methyl and every glycerol formyl esters is assessed. The analysis of FAME present in any oily sample is conducted by dissolving 1.00 g of the sample directly in 10mL of heptane and 10mL of the 2 w/v. % internal standard solution followed.

1 μL of the solution of 50mg/mL of FAME/FAGE or FAME mixture, were analyzed in a Shimadzu 2100, gas chromatograph fitted with a Zebron ZB-5MS GC capillary column 30 m x 0.25 mm x 0.25 μm. Injection temperature of 320 °C, split ration 50:1 and FID temperature at 320°C, as auxiliary gas $N_2$ was used with a flow of 30mL/min, in addition to $H_2$ 40mL/min and air 400mL/min. Total column flow 1mL/min. linear velocity 25.1cm/s. Carrier gas pressure (Helium) 80.9kPa.

The ramp employed was the following: T initial= 40° C held for 1 minute, subsequently rising at 10° C /min. to 150° C then held for 3 min., followed by two sequential ramps of 5° C/min. each, first to 200° C (held for 5mins.), second and finally to 320° C (held for 5 minutes), with a total run of 59 minutes.

Samples of 50 μl were diluted in 950 μl of the internal standard solution of methyl heptadecanoate prepared in heptane, and 1 μl was injected in a split ratio of 1:50. The conversion rate (%) was calculated with Eq. (1):

$$\boldsymbol{conversion\ rate}\ (\%) = \frac{\frac{\sum \mathbf{fage}}{\mathbf{Amhd\ x\ Fr}}}{\frac{\sum \mathbf{fage}}{\mathbf{Amhd\ x\ Fr}} + \frac{\sum \mathbf{fame}}{\mathbf{Amhd}}}\ \boldsymbol{x}\ \mathbf{100} \qquad \mathbf{(1)}$$

where, **Σfage** is the sum of all FAGE peak areas; **Amhd** is peak area for methyl heptadecanoate (internal standard); **Σfame** is the sum of all FAME peak areas, and **Fr** is the response factor.

As shown in Table 5 below, most of lipases rarely produced the FAGE compound, while Novozym 435 (*Candida antarctica*) showed the higher catalytic activity.

Table 5. Lipase-catalyzed synthesis of FAGE[a]

| **Source** | **Regioselecti ve** | **Conversion[b] (%)** |
|---|---|---|
| *Pseudomonas fluorescens* | Non-selective | ND |
| *Thermomyces lanuginosus* | *sn*-1,3 | 11.14 |
| *Mucor miehei* | *sn*-1,3 | 2.28 |

(continued)

| Source | Regioselecti ve | Conversion[b] (%) |
|---|---|---|
| *Rhizopus oryzae* | *sn*-1,3 | ND |
| *Candida rugosa* | Non-selective | ND |
| *Candida antárctica* | Non-selective | 40.85 |
| *Pseudomonas cepacia* | Non-selective | ND |
| *Rhizopus niveus* | *sn*-1,3 | ND |
| *Rhizopus arrhizus* | *sn*-1,3 | ND |
| *Penicillium camemberti* | Non-selective | ND |
| *Aspergillus oryzae* | *sn*-1,3 | ND |
| *Lipoprotein-lipase from Pseudomonas sp.* | Non-selective | ND |
| *Lipoprotein-lipase from Burkholderia sp.* | Non-selective | ND |
| *Lipase from porcine páncreas* | *sn*-1,3 | ND |
| *Lipase from Proteus sp.* K107 | Non-selective | 5.52 |

[a] Reaction conditions: FAME (2.7 mmol), GF (2.3 mmmol), water (50 ul); and lipases (0.03 g) were mixed using a magnetic stirrer (200 rpm), at 40 °C for 12 h.
[b] Determined by GC.

Example 6: Synthesis of FAME/FAGE with a blend of *Candida antarctica* lipase (Novozym 453) and *Thermomyces lanuginosus* lipase (Lypozyme TL IM)

The following proportions of Lypozyme TL IM and Novozym 435 were added in a round bottom flask to made up 10wt.% to 2.5g (8.44 mmol) of FAME (<500ppm moisture):

Table 6

| Trial | Catalyst (wt.%) |
|---|---|
| | |
| 1 | 0%Novozym 435;10% Lypozyme TL IM |
| 2 | 0.1%Novozym 435;9.9% Lypozyme TL IM |
| 3 | 1%Novozym 435; 9% Lypozyme TL IM |
| 4 | 5%Novozym 435; 5% Lypozyme TL IM |
| 5 | 10%Novozym 435; 0% Lypozyme TL IM |

The mixture starts a relax with agitation (200rpm) at 50°C with a continuous flow of $N_2$ for an initial time of 30mins. Glycerol formal (<1000ppm moisture), was sequentially added drop wise at a rate of 100μl after every 5 minutes for a total period of 45 minutes. After the addition time the reflux under nitrogen was kept for additional 30 minutes.

Apparent conversion of FAME to FAGE was qualitatively evaluated by TLC with the following results:

**Conversion 1< Conversion 2< Conversion 3< Conversion 5< Conversion 4**

These results are indicative that *Candida antarctica* lipase provides higher conversion rates than *Thermomyces lanuginosus* lipase, when used alone or with small proportion of *Thermomyces lanuginosus* lipase but at the same time reveals a synergistic effect in conversion when used in combination at a balanced proportion.

**Claims**

**1.** A process for preparing a biofuel composition comprising the steps of:

a) reacting a fatty acid alkyl ester of formula (II) or a source of fatty acid alkyl esters of formula (II) with glycerol formal in the presence of an immobilised lipolytic enzyme,

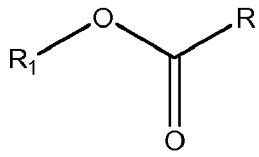

**(II)**

to obtain fatty acid glycerol formal (FAGE) of formula (I):

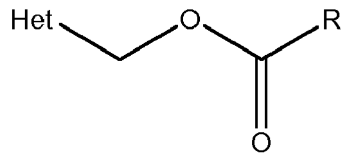

**(I)**

b) neutralizing excess fatty acids to acid index below 0.5mg KOH/g of sample.
c) adding a fatty acid alkyl ester of formula (II) to obtain a ratio of fatty acid alkyl esters /FAGE from 60/40 to 90/10 wt.%;
d) adding an acetal mixture of glycerol formal/ methoxy methyl glycerol formal (GFoMoM) in a ratio from 1 to 5 v/v% respect to the total volume of fatty acid alkyl esters /FAGE resulting an ester/acetal mixture;
e) adding hydrotreated vegetable oil (HVO) to obtain a ratio (%v/v), HVO/the ester/acetal mixture obtained in d) from 60/40 to 90/10;

where

**Het** is selected from;

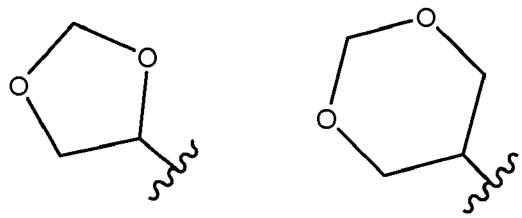

**R** $C_1$-$C_{28}$ alkyl radical or akylen radical $C_1$-$C_{28}$ ; y
**$R_1$** $C_1$-$C_6$ alkyl radical.

**2.** The process according to Claim 1 where the fatty acid akyl ester is a fatty acid methyl ester (FAME) of formula :

**3.**

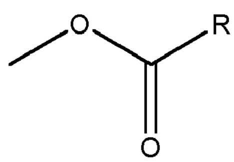

**(IIa)**

where R is defined in claim 1.

4. The process according to any of the previous claims, **characterized by** FAGE is derived from animal fats, rapeseed oil, food waste oil, used cooking oil, sunflower oil, palm oil, coconut oil, soy oil, olive oil, industrial residues and mixtures thereof.

5. The process according to any of the previous claims **characterized by** the lipolytic enzyme is selected from: *Thermomyces lanuginosus* lipase, *Candida antarctica* lipase B, *Proteus sp.* K107 lipase or a mixture thereof.

6. The process according to any of the previous claims **characterized by** comprising a step previous to step d) of mixing an acetal mixture glycerol formal/GFoMoM between 50/50 to 90/10 wt.% with an alkali metal hydride dissolved in a solvent and filtering to obtain an acetal mixture glycerol formal/GFoMoM between 30/70 to 10/90 wt.%.

7. The process according to any of the previous claims **characterized by** comprising the addition of additives selected from the group of: antioxidants, cold flow improvers and/or antifoaming agents in step d) or after step d).

8. A biofuel composition obtainable by the process of any of claim 1-6.

9. The biofuel composition according to the claim 7 **characterized by** a density value between 765 kg/$m^3$-845 kg/$m^3$ measured by EN ISO 3675.

10. The biofuel composition according to claim 7 and 8 **characterized by** a kinematic viscosity value between 2 $mm^2$/s-4.5 $mm^2$/s measured by EN ISO 3104.

11. The biofuel composition according to claim 7 to 9 **characterized by** a cold filter plugging point lower than 5°C measured by EN 116.

12. Use of the biofuel composition according any of the claims 7 to 10 as fuel for diesel engines.

Europäisches Patentamt
European Patent Office
Office européen des brevets

# EUROPEAN SEARCH REPORT

Application Number
EP 23 38 3360

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HERNÁNDEZ JUAN J ET AL: "Performance and regulated gaseous emissions of a Euro 6 diesel vehicle with Lean NOx Trap at different ambient conditions: Sensitivity to the type of fuel", ENERGY CONVERSION AND MANAGEMENT, ELSEVIER SCIENCE PUBLISHERS, OXFORD, GB, vol. 219, 8 June 2020 (2020-06-08), XP086207106, ISSN: 0196-8904, DOI: 10.1016/J.ENCONMAN.2020.113023 [retrieved on 2020-06-08] * abstract * * page 2, left-hand column * * paragraph [2.2.] * * table 3 * ----- | 1,2,4-12 | INV. C12P7/649 C10L1/02 C12P7/6458 C12P17/04 C12P17/06 |
| Y | LAPUERTA MAGÍN ET AL: "Properties of fatty acid glycerol formal ester (FAGE) for use as a component in blends for diesel engines", BIOMASS AND BIOENERGY, vol. 76, 1 May 2015 (2015-05-01), pages 130-140, XP093039261, AMSTERDAM, NL ISSN: 0961-9534, DOI: 10.1016/j.biombioe.2015.03.008 Retrieved from the Internet: URL:https://www.sciencedirect.com/science/article/pii/S0961953415000884/pdfft?md5=7678a803c82b428a2360832b77184e7a&pid=1-s2.0-S0961953415000884-main.pdf> * abstract * * pages 132-133 * * figures 2, 3, 5 * ----- | 1,2,4-12 | TECHNICAL FIELDS SEARCHED (IPC) C12P C10L |

-/--

The present search report has been drawn up for all claims

2

| Place of search | Date of completion of the search | Examiner |
|---|---|---|
| Berlin | 14 June 2024 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches Patentamt
European Patent Office
Office européen des brevets

# EUROPEAN SEARCH REPORT

Application Number
EP 23 38 3360

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| Y | RODRÍGUEZ-FERNÁNDEZ JOSÉ ET AL: "Selection of Blends of Diesel Fuel and Advanced Biofuels Based on Their Physical and Thermochemical Properties", ENERGIES, vol. 12, no. 11, 28 May 2019 (2019-05-28), page 2034, XP093039272, DOI: 10.3390/en12112034 Retrieved from the Internet: URL:https://www.sciencedirect.com/science/article/pii/S0016236119300687/pdfft?md5=423e1e1a0ab80154f37677eae0b8cd1e&pid=1-s2.0-S0016236119300687-main.pdf> * abstract; figures 1, 4, 5 * * page 2 * * paragraph [4.3.] * ----- | 1,2,4-12 | |
| A | VÄNTTINEN E ET AL: "Optically Active 1,3-Dioxolane-4-methanols. Lipase-Catalysed Acylation of Racemic Ketals and Diastereomeric Acetals", TETRAHEDRON ASYMMETRY, PERGAMON PRESS LTD, OXFORD, GB, vol. 7, no. 10, 1 October 1996 (1996-10-01), pages 3037-3046, XP004048392, ISSN: 0957-4166, DOI: 10.1016/0957-4166(96)00394-1 * abstract * * table 1; compound 2 * * page 3039 * ----- | 1,2,4,5 | TECHNICAL FIELDS SEARCHED (IPC) |

-/--

The present search report has been drawn up for all claims

2

| Place of search | Date of completion of the search | Examiner |
| --- | --- | --- |
| Berlin | 14 June 2024 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 38 3360

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ITABAIANA IVALDO ET AL: "Continuous flow valorization of fatty acid waste using silica-immobilized lipases", GREEN CHEMISTRY, vol. 15, no. 2, 1 January 2013 (2013-01-01), pages 518-524, XP093174604, GB ISSN: 1463-9262, DOI: 10.1039/c2gc36674f * abstract * * paragraph [3.2.]; table 1 * * paragraph [2.1.] * ----- | 1,2,4,5 | |
| A | WO 2014/102796 A1 (TRANS BIO DIESEL LTD [IL]) 3 July 2014 (2014-07-03) * claims; examples * ----- | 5 | |
| A | RODRÍGUEZ-FERNÁNDEZ JOSÉ ET AL: "Regeneration of diesel particulate filters: Effect of renewable fuels", RENEWABLE ENERGY, PERGAMON PRESS, OXFORD, GB, vol. 104, 30 November 2016 (2016-11-30), pages 30-39, XP029874513, ISSN: 0960-1481, DOI: 10.1016/J.RENENE.2016.11.059 * abstract; figure 8 * ----- | 1,7-12 | TECHNICAL FIELDS SEARCHED (IPC) |

-/--

The present search report has been drawn up for all claims

2

| Place of search | Date of completion of the search | Examiner |
|---|---|---|
| Berlin | 14 June 2024 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 38 3360

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LAPUERTA MAGÍN ET AL: "Improvement of cold flow properties of a new biofuel derived from glycerol", FUEL, vol. 242, 1 April 2019 (2019-04-01), pages 794-803, XP093039262, GB ISSN: 0016-2361, DOI: 10.1016/j.fuel.2019.01.066 Retrieved from the Internet: URL:https://www.sciencedirect.com/science/article/pii/S0016236119300687/pdfft?md5=423e1e1a0ab80154f37677eae0b8cd1e&pid=1-s2.0-S0016236119300687-main.pdf> * abstract; figures 2-9 * ----- | 1,7-12 | |
| A | LAPUERTA MAGÍN ET AL: "Optimization of a diesel engine calibration for operating with a residual glycerol-derived biofuel", INTERNATIONAL JOURNAL OF ENGINE RESEARCH, vol. 22, no. 4, 1 April 2021 (2021-04-01), pages 1273-1284, XP093039150, GB ISSN: 1468-0874, DOI: 10.1177/1468087419891535 Retrieved from the Internet: URL:http://journals.sagepub.com/doi/full-xml/10.1177/1468087419891535> * abstract * * page 1275 - page 1276 * ----- | 1,7-12 | TECHNICAL FIELDS SEARCHED (IPC) |

The present search report has been drawn up for all claims

2

| Place of search | Date of completion of the search | Examiner |
|---|---|---|
| Berlin | 14 June 2024 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 38 3360

This annex lists the patent family members relating to the patent documents cited in the above-mentioned European search report. The members are as contained in the European Patent Office EDP file on
The European Patent Office is in no way liable for these particulars which are merely given for the purpose of information.

14-06-2024

| Patent document cited in search report | Publication date | Patent family member(s) | Publication date |
|---|---|---|---|
| WO 2014102796 A1 | 03-07-2014 | AR 094350 A1 | 29-07-2015 |
| | | AU 2013368822 A1 | 09-07-2015 |
| | | CA 2896428 A1 | 03-07-2014 |
| | | CL 2015001836 A1 | 27-11-2015 |
| | | CN 104884630 A | 02-09-2015 |
| | | EP 2938739 A1 | 04-11-2015 |
| | | HK 1211987 A1 | 03-06-2016 |
| | | JP 6430960 B2 | 28-11-2018 |
| | | JP 2016503652 A | 08-02-2016 |
| | | KR 20150102034 A | 04-09-2015 |
| | | PE 20151447 A1 | 14-10-2015 |
| | | RU 2015126914 A | 02-02-2017 |
| | | SG 11201505110Y A | 30-07-2015 |
| | | TW 201432050 A | 16-08-2014 |
| | | US 2014186908 A1 | 03-07-2014 |
| | | US 2015353970 A1 | 10-12-2015 |
| | | US 2017166850 A1 | 15-06-2017 |
| | | WO 2014102796 A1 | 03-07-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2730567 A
- EP 2049623 A
- WO 2014102796 A


### Non-patent literature cited in the description


- *CHEMICAL ABSTRACTS*, 99569-11-6